(19) Europäisches Patentamt
European Patent Office
Office européen des brevets

(11) Veröffentlichungsnummer: **0 239 667 B1**

(12) # EUROPÄISCHE PATENTSCHRIFT

(45) Veröffentlichungstag der Patentschrift: **02.05.91**

(51) Int. Cl.5: **C07D 471/04**, A61K 31/435, //(C07D471/04,221:00,209:00)

(21) Anmeldenummer: **86115006.8**

(22) Anmeldetag: **29.10.86**

Die Akte enthält technische Angaben, die nach dem Eingang der Anmeldung eingereicht wurden und die nicht in dieser Patentschrift enthalten sind.

(54) **5- oder 6-substituierte-beta-carbolin-3-carbonsäureester.**

(30) Priorität: **20.03.86 DE 3609699**

(43) Veröffentlichungstag der Anmeldung:
**07.10.87 Patentblatt 87/41**

(45) Bekanntmachung des Hinweises auf die Patenterteilung:
**02.05.91 Patentblatt 91/18**

(84) Benannte Vertragsstaaten:
**AT BE CH DE ES FR GB GR IT LI LU NL SE**

(56) Entgegenhaltungen:
**EP-A- 0 054 507**
**EP-A- 0 130 140**
**EP-A- 0 190 987**
**DE-A- 3 322 895**

(73) Patentinhaber: **SCHERING AKTIENGESELLSCHAFT Berlin und Bergkamen**
**Müllerstrasse 170/178 Postfach 65 03 11**
**W-1000 Berlin 65(DE)**

(72) Erfinder: **Honoré, Tage, Dr.**
**Ganloeseparken 42**
**DK-2760 Maaloev(DK)**
Erfinder: **Biere, Helmut, Dr.**
**Zeltinger Strasse 15**
**W-1000 Berlin 28(DE)**
Erfinder: **Huth, Andreas, Dr.**
**Kirchweg 55**
**W-1000 Berlin 38(DE)**
Erfinder: **Rahtz, Dieter, Dr.**
**Krottnaurerstrasse 24a**
**W-1000 Berlin 38(DE)**
Erfinder: **Schmiechen, Ralph, Dr.**
**Bayernring 27**
**W-1000 Berlin 42(DE)**
Erfinder: **Seidelmann., Dieter, Dr.**
**Stierstrasse 14**
**W-1000 Berlin 41(DE)**
Erfinder: **Kehr, Wolfgang, Dr.**
**Biedermannweg 11**
**W-1000 Berlin 19(DE)**

**EP 0 239 667 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Erfinder: **Schneider, Herbert Hans, Dr.**
**Duisburger Strasse 20**
**W-1000 Berlin 15(DE)**
Erfinder: **Engelstoft, Mogens, Dr.**
**Mosegard Park 121**
**DK-3500 Vaerlose(DK)**
Erfinder: **Hansen, Bondo John, Dr.**
**Havretoften 10**
**DK-2800 Lyngby(DK)**
Erfinder: **Wätjen, Frank, Dr.**
**Jostiens Vej 27**
**DK-2880 Bajsvaerd(DK)**

## Beschreibung

Die Erfindung betrifft neue 5- oder 6-substituierte β-Carbolin-3-carbonsäureester.

In zahlreichen Patenten wurden β-Carbolin-3-carbonsäureester beschrieben wie beispielsweise in dem EP-Patent 30254 der β-Carbolin-3-carbonsäure-isopropylester und der β-Carbolin-4-ethyl-3-carbonsäure-isopropylester, in der DE-OS 3322895 der 5-[1-(4-Chlorphenyl)-ethoxy-4-methoxymethyl-β-carbolin-3-oarbonsäure-isopropylester und in dem US-Patent 4435403 der 5-Benzyloxy- und der 6-Benzyloxy-4-methoxymethyl-β-carbolin-3-carbonsäureethylester.

Diesen Patentschriften kann man entnehmen, daß β-Carbolin-3-carbonsäureester das Zentralnervensystem beeinflussen und als Psychopharmaka geeignet sind.

β-Carbolin-3-carbonsäureethylester werden durch entsprechende Enzyme relativ leicht in die entsprechenden Säuren gespalten, die keine oder eine geringe Affinität zu Benzodiazepinrezeptoren aufweisen. Es wurde nun überraschenderweise gefunden, daß die erfindungsgemäßen Verbindungen diesen Nachteil nicht besitzen, sondern eine erhöhte Stabilität gegen Esterasen besitzen.

Die erfindungsgemäßen 5- oder 6-substituierten β-Carbolin-3-carbonsäureester haben die allgemeine Formel I

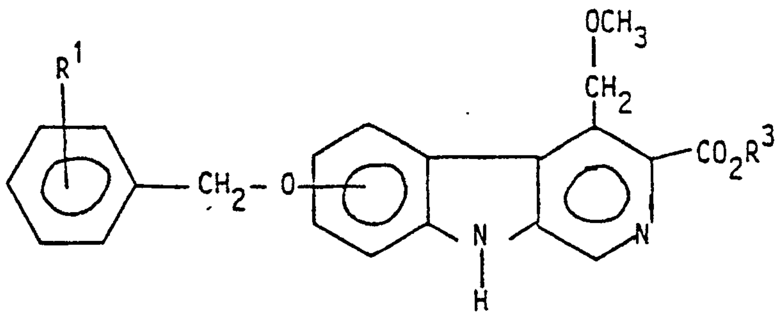

I

worin

$R^1$ Wasserstoff, Nitrilo, Halogen, $C_{1-4}$-Alkyl, $C_{1-4}$-Alkoxy bedeutet und

$R^3$ eine verzweigte $C_{3-6}$-Alkylgruppe ist, die gegebenenfalls mit Halogen substituiert ist oder eine $C_{3-6}$-Cycloalkylgrüppe, die gegebenenfalls methylsubstituiert ist und

$R^1$ ein- oder mehrfach stehen kann.

Unter Halogen ist beispielsweise Fluor, Chlor oder Brom zu verstehen, wobei Fluor und Chlor bevorzugt sind.

Geeignete niedere Alkyl- und niedere Alkoxygruppen $R^1$ mit 1 - 4 Kohlenstoffatomen beispielsweise Methyl, Ethyl, H sind Propyl, Isopropyl, Butyl, Isobutyl, 2-Butyl, tert.-Butyl, wobei niedere Alkylgruppen mit 1 - 2 Kohlenstoffatomen bevorzugt sind.

Der Substituent $R^1$ kann in 2-, 3- oder 4-Stellung am Phenylrest stehen, der ein- oder mehrfach substituiert sein kann, bevorzugt ein- oder zweifach.

Als verzweigte Alkylgruppen mit 3 - 6 Kohlenstoffatomen $R^3$ seien beispielsweise die folgenden sekundären und tertiären Alkylgruppen genannt: Isopropyl, tert.-Butyl, Isobutyl, 2-Butyl, Neopentyl u.a..

Insbesondere geeignet sind verzweigte Alkylgruppen mit 3-4 Kohlenstoffatomen.

Als $C_{3-6}$-Cycloalkylgruppen $R^3$, die gegebenenfalls methylsubstituiert sein können, seien beispielsweise genannt: Cyclopropyl, Methylcyclopropyl, Cyclobutyl, Cyclopentyl, Methylcyclopentyl, Cyclohexyl u.a.

Die Wirkung der Verbindungen der allgemeinen Formel I auf das Zentralnervensystem und die Stabilität gegen Esterasen wurde durch Untersuchungen nach an sich bekannten Methoden bestimmt.

Wie der folgenden Tabelle am Beispiel des 6-Benzyloxy-4-methoxymethyl-β-carbolin-3-carbonsäure-isopropylester (B) im Vergleich zum bekannten 6-Benzyloxy-4-methoxymethyl-β-carbolin-3-carbonsäureethylester (A) zu entnehmen ist, zeigen die erfindungsgemäßen Verbindungen überlegene Wirkungen im in-vivo-Bindungstest, im Kamintest und in der Bioverfügbarkeit.

| ED$_{50}$ mg/kg in vivo | Kamintest ED$_{50}$ (mg/kg) i.p. | Bioverfügbarkeit Ratte % |
|---|---|---|
| A 5,6 | 12,1 | 17 |
| B 0,4 | > 50 | 32 |

Der ED₅₀-Wert stellt die Dosis einer Versuchssubstanz dar, die eine Reduktion der spezifischen Bindung des Flunitrazepams an dem Benzodiazepin-Rezeptor in einem lebenden Gehirn auf 50 % des Kontrollwertes bewirkt.

Der Kamintest wurde nach der Methode von Poissier Jr. et al. Med. exp. 3 1960, Seite 81-84, durchgeführt.

Die oben genannten bekannten β-Carbolin-3-carbonsäureisopropylester besitzen im Vergleich zu den erfindungsgemäßen Verbindungen ein wesentlich schlechteres Bindungsvermögen an die Benzodiazepin-Rezeptoren.

Die Verbindungen der allgemeinen Formel I zeigen im pharmakologischen Test überlegene psychotrope Eigenschaften und insbesondere überlegene anxiolytische Eigenschaften, wobei eine weniger sedative Wirkung festgestellt werden kann.

Die erfindungsgemäßen Verbindungen sind aufgrund ihrer wertvollen pharmakologischen Eigenschaften, insbesondere ihrer Wirkung auf das Zentralnervensystem, als Psychopharmaka in der Humanmedizin geeignet.

Die Verbindungen können besonders zur Behandlung von Angst, Epilepsie und Schlafstörungen verwendet werden.

Die erfindungsgemäßen Verbindungen können zur Formulierung von pharmazeutischen Präparationen, zum Beispiel für die orale und parentale Anwendung nach an sich bekannten Methoden der Galenik verwendet werden.

Als Hilfsstoffe zur Formulierung von pharmazeutischen Präparationen sind solche physiologisch verträglichen organischen und anorganischen Trägersubstanzen für die enterale und parenterale Anwendung geeignet, die gegenüber den erfindungsgemäßen Verbindungen inert sind.

Als Trägersubstanzen seien beispielsweise genannt: Wasser, Salzlösungen, Alkohole, Polyäthylenglykole, polyhydroxyethoxyliertes Rizinusöl, Gelatine, Lactose, Amylose, Magnesiumstearat, Talkum, Kieselsäure, Fettsäuremono-und diglyceride, Pentaerythritolfettsäureester, Hydroxymethylcellulose und Polyvinylpyrrolidon.

Die pharmazeutischen Präparationen können sterilisiert und/oder mit Hilfsstoffen, wie Schmiermitteln, Konservierungsstoffen, Stabilisatoren, Netzmitteln, Emulgatoren, Puffermitteln und Farbstoffen, versetzt werden.

Für die parenterale Anwendung sind insbesondere Injektionslösungen oder Suspensionen, insbesondere wässrige Lösungen der aktiven Verbindungen in polyhydroxyethoxyliertem Rizinusöl, geeignet. Es können aber auch physiologisch verträgliche grenzflächen-aktive Hilfsstoffe wie Salze der Gallensäuren oder tierische und pflanzliche Phospholipide aber auch Mischungen davon verwendet werden, sowie Liposome oder deren Bestandteile als Trägersysteme verwendet werden.

Für die orale Anwendung sind insbesondere Tabletten, Dragees oder Kapseln mit Talkum und/oder Kohlenwasserstoffträger oder -binder, wie zum Beispiel Lactose, Mais- oder Kartoffelstärke, geeignet. Die Anwendung kann auch in flüssiger Form erfolgen, wie zum Beispiel als Saft, dem gegebenenfalls ein Süßstoff beigefügt wird.

Die erfindungsgemäßen Verbindungen werden in einer Dosiseinheit von 0,05 bis 100 mg aktiver Substanz in einem physiologisch verträglichen Träger eingebracht.

Die erfindungsgemäßen Verbindungen werden in einer Dosis von 0,1 bis 300 mg/Tag, jvorzugsweise 1 bis 30 mg/Tag, angewendet.

Die Herstellung der erfindungsgemäßen Verbindungen erfolgt nach an sich bekannten Methoden.

Beispielsweise erfolgt die Herstellung der Verbindungen der allgemeinen Formel I dadurch, daß man

a) eine Verbindung der allgemeinen Formel II

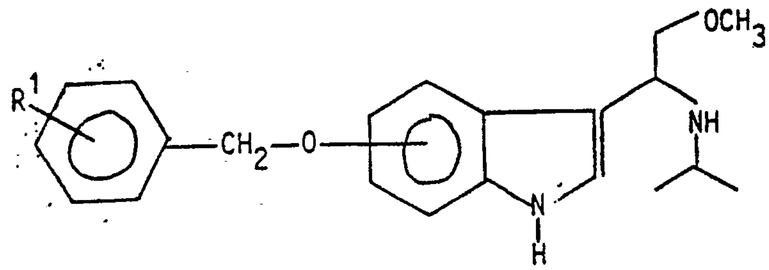

II,

worin
$R^1$ die oben genannte Bedeutung hat, mit dem entsprechenden Ester eines Glycinimins der allgemeinen Formel III

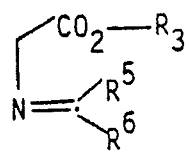

III,

worin
$R^3$ die oben genannte Bedeutung hat, worin $R^5$ ein vorzugweise durch 4-Methoxy oder Wasserstoff substituierter Aromat und $R^6$ Wasserstoff oder gegebenenfalls ein Aromat ist, umsetzt und anschließend das Imin hydrolytisch zum Amin der allgemeinen Formel IV

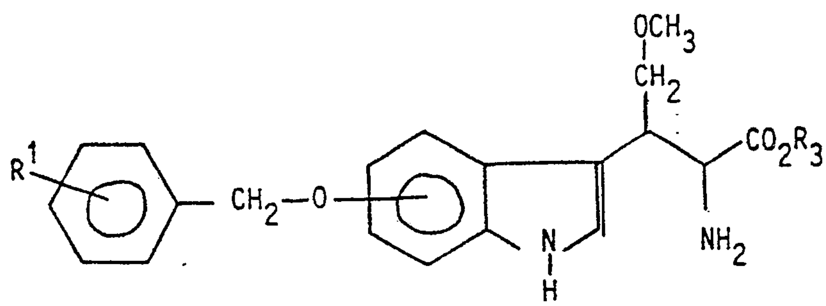

IV,

spaltet, mit Formaldehyd oder Glyoxylsäure cyclisiert, wobei die Cyclisierung auch grundsätzlich mit der sauren Spaltung des Imins als Eintopfreaktion verbunden werden kann, und anschließend aromatisiert und gewünschtenfalls die Benzylgruppe abspaltet und die so erhaltene frei Hydroxygruppe anschließend veräthert mit = einer Verbindung der allgemeinen Formel V

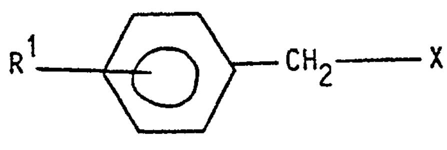

V,

worin
$R^1$ die oben genannte Bedeutung hat und X Halogen, Mesyl oder Tosyl bedeutet oder
b) eine Verbindung der allgemeinen Formel VI

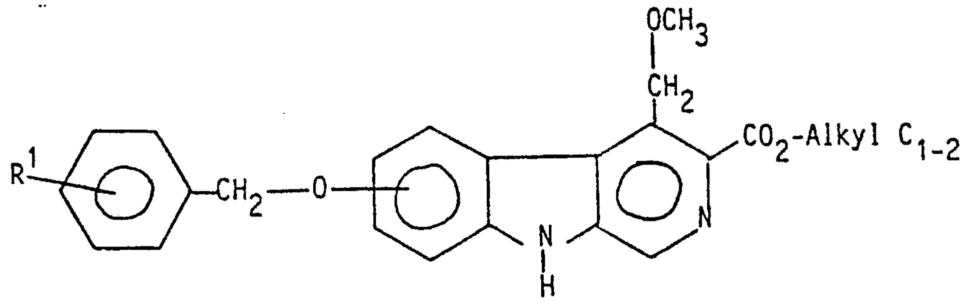

VI,

worin $R^1$ die oben genannte Bedeutung hat, umestert.

Die Substitution nach Verfahrensvariante a) wird beispielweise vorgenommen, indem die Verbindung der allgemeinen Formel II in einem polaren Lösungsmittel - vorzugsweise aprotisch wie zum Beispiels Dimethylformamid oder N-Methylpyrrolidon gegebenenfalls unter Zusatz eines anderen Lösungsmittels wie beispielsweise Toluol zu einem Gemisch des Imins der allgemeinen Formel III im gleichen Lösungsmittel mit einer Base, vorzugsweise Kaliumcarbonat, bei erhöhter Temperatur, vorzugsweise bei 90 - 105 °C, gibt und bei der gleichen Temperatur umsetzt.

Die anschließende Spaltung des gebildeten Imins zum Amin kann durch Hydrolyse, vorzugsweise im sauren Bereich, durchgeführt werden.

Die Cyclisierung nach Verfahrensvariante a) wird beispielsweise vorgenommen, indem man die Verbindung der allgemeinen Formel IV in einem inerten mit Wasser nicht mischbaren Lösungsmittel wie Benzol, Toluol, Xylol, Chlorbenzol, Anisol, Mesitylen löst und mit Paraformaldehyd gegebenenfalls bei erhöhter Temperatur bis zur Siedetemperatur des Lösungsmittels umsetzt. Die Umsetzung mit Formaldehyd kann auch in wässriger Lösung bei Raumtemperatur und bei pH 2 - 7 vorgenommen werden, wenn der Paraformaldehyd vorher in Gegenwart von Säuren bei erhöhter Temperatur in wässriger Lösung zum Formaldehyd gespalten wurde.

Die Cyclisierung kann auch mit Glyoxylsäure erfolgen. Hierbei versetzt man zweckmäßigerweise das Amin, das in Wasser oder in einem inerten organischen Lösungsmittel wie beispielsweise Essigester gelöst ist, mit einer wässrigen Lösung von Glyoxylsäure bei einem pH-Wert von 0 - 7, vorzugsweise 4. Die anschließende Decarboxylierung wird bei erhöhter Temperatur, gegebenenfalls bei der Siedetemperatur eines oben genannten inerten Lösungsmittels, zum Beispiel Toluol oder Xylol, vorgenommen.

Bei der Cyclisierung entsteht ein 1,2,3,4-Tetrahydro-9H-Pyrido-[3,4-b]-indolderivat, das anschließend in beiden Fällen dehydriert wird.

Die Behydrierung kann beispielsweise so durchgeführt werden, daß man das Ausgangsmaterial in einem inerten Lösungsmittel löst bzw. suspendiert und elementaren Schwefel hinzugibt, dessen Menge in etwa so bemessen ist, daß pro Doppelbindung ein Moläquivalent Schwefel verwendet wird. Das Reaktionsgemisch wird mehrere Stunden am Rückfluß gekocht, wobei der Reaktionsverlauf dünnschichtchromatographisch verfolgt wird. Alle aprotischen Lösungsmittel, deren Siedepunkt über 100 °C liegt und die gegenüber dem Ausgangsmaterial inert sind, wie beispielsweise Xylol, Mesitylen, Anisol, Toluol, Chlorbenzol und Diphenyläther sind für die Dehydrierung geeignet.

Eine weitere Methode ist die Dehydrierung mit Edelmetallkatalysatoren wie Platin in feinverteilter Form, Palladium-Mohr oder Palladium-Kohle in Xylol, Mesitylen oder Cumol bei 120 - 180 °C und Reaktionszeiten von 2 - 6 Stunden.

Eine andere bevorzugte Methode ist die Dehydrierung mit tert.-Butylhypochlorit und tertiären Basen bevorzugt im Bereich von -15 °C bis Raumtemperatur (Deutsche Patentanmeldung Nr. 3504045.9).

Die Abspaltung der Benzylgruppe erfolgt beispielsweise durch Hydrierung in Gegenwart eines Katalysators wie beispielsweise eines Edelmetallkatalysators wie Palladium auf einem geeigneten Träger wie Kohle oder mit Raney-Nickel in protischen Lösungsmitteln wie beispielsweise Alkoholen bei Wasserstoffnormaldruck oder erhöhtem Wasserstoffdruck.

Die Reaktionstemperaturen liegen von Raumtemperatur bis zur Siedetemperatur des Lösungsmittels. Im allgemeinen ist die Reaktion nach 2 - 10 Stunden beendet.

Die Verätherung der 5- oder 6-Hydroxy-β-carbolinderivate erfolgt z.B. in Gegenwart von Basen mit einem Benzylderivat der allgemeinen Formel V mit einer Fluchtgruppe wie beispielsweise Halogenid, Tosylat oder Mesylat in polaren Lösungsmitteln wie beispielsweise Dimethylsulfoxid, Dimethylformamid, Acetonitril oder Ethanol bei Temperaturen bis zum Siedepunkt des Lösungsmittels.

Als Basen sind beispielsweise geeignet: Alkaliverbindungen wie zum Beispiel Natrium- oder Kaliumhydroxyde, -carbonate, -alkoholate oder -hydride, Kaliumfluorid- DBU, DABCO oder Hünigbase. Gegebenenfalls kann auch in Gegenwart von Phasentransferkatalysatoren wie beispielsweise Kronenäther, Aliquat,

Tetrabutylammoniumhydrogensulfat oder 2,2,2-Kryptand gearbeitet werden.

Zweckmäßigerweise wird die Umsetzung unter Inertgasatmosphäre beispielsweise unter Argon oder Stickstoff vorgenommen.

Für die Umesterung nach Verfahrensvariante b) sind alle gebräuchlichen Methoden geeignet wie beispielsweise die Umsetzung mit dem entsprechenden Alkohol oder Alkalialkoholat, gegebenenfalls kann man Titantetra-isopropylat als Katalysator oder äquimolar bis zu einem Überschuß im wasserfreien entsprechenden Alkohol zufügen. Üblicherweise wird die Umesterung bei Temperaturen von 60 bis 120 °C durchgeführt und ist nach ca. 2 - 6 Stunden beendet.

Grundsätzlich sind auch Umesterungen mit den folgenden Reagenzien möglich: Triphenylphosphin/Azodicarbonsäureester oder Bromtribromid/Alkohol oder Kupfersalze/Alkohol u.a..

Die Einführung der tert.-Butylestergruppe erfolgt insbesondere beispielsweise durch Umsetzung der Carbonsäure mit tert.-Butoxy-bis-dimethyl-aminomethan. Im allgemeinen wird die Reaktion unter Inertgasatmosphäre wie Argon oder Stickstoff und unter Feuchtigkeitsausschluß bei erhöhter Temperatur durchgeführt.

Die Verseifung der Estergruppe kann sauer oder alkalisch erfolgen; vorzugsweise wird alkalisch verseift, indem der Ester mit verdünnter wässriger Alkalilauge wie Kalium-oder Natriumhydroxid, in einem protischen Lösungsmittel, wie zum Beispiel Methanol, Ethanol oder Ethylenglykol, auf Temperaturen bis zur Rückflußtemperatur des Reaktionsgemisches erhitzt wird.

Bei anfallenden Razematen kann eine Razematabspaltung nach gebräuchlichen Methoden vorgenommen werden.

Die Herstellung der Ausgangsverbindungen ist bekannt oder erfolgt nach bekannten Verfahren.

Die nachfolgenden Beispiele sollen das erfindungsgemäße Verfahren erläutern.

Beispiel 1

A)

6 g (43,5 mMol) fein pulverisiertes Kaliumcarbonat werden in 25 ml absolutem Dimethylformamid bei 95 °C unter Stickstoff 10 Minuten gerührt. Anschließend werden in der Wärme 10 g (29,6 mMol) 5-Benzyloxy-3-(1-isopropylamino-2-methoxyethyl)-indol zugefügt und ungefähr 10 Minuten bei 95 °C bis zu dessen Auflösung gerührt. Daraufhin wird ebenfalls bei 95 °C eine Lösung von 34,5 mMol Glycinanisaldehydisopropylester in 25 ml Dimethylformamid tropfenweise in einem Zeitraum von 30 Minuten zugesetzt. Die Lösung wird solange gerührt bis das Ausgangsindol im Dünnschichtchromatogramm nicht mehr nachweisbar ist. Nach Abkühlen wird vom Kaliumkarbonat abgesaugt und mit Toluol nachgewaschen. Nach Zugabe von 100 ml Toluol werden 200 ml 1N-Salzsäure zugesetzt und 3 Stunden bei Raumtemperatur gerührt. Die Toluolphase wird abgetrennt und die wässrige saure Phase mit 100 ml Toluol ausgeschüttelt. Die organische Phase wird verworfen. Die saure Phase wird auf 5 °C gekühlt mit 100 ml Toluol versetzt und mit 4N-Natronlauge auf einen pH von 10 bis 12 eingestellt . Nach Ausschütteln wird nochmals mit 100 ml Toluol ausgeschüttelt und die vereinigte organische Phase mit 50 ml Wasser gewaschen, getrocknet, filtriert und eingeengt.

Man erhält 70 % 2-Amino-3-(5-benzyloxyindol-3-yl)-4-methoxy-buttersäureisopropylester als Öl.

B)

3,8 g 2-Amino-3-(5-benzyloxyindol-3-yl)-4-methoxybuttersäureisopropylester (10 ml Mol) werden in 80 ml Xylol gelöst und zu einer 45 Minuten auf 100 °C erwärmten Suspension von 360 mg Paraformaldehyd in 60 ml Xylol getropft. Die Mischung wird dann 2 Stunden am Wasserabscheider zum Rückfluß erhitzt. Nach Einengen wird der Rückstand über Kieselgel mit Methylenchlorid : Aceton = 1 : 1 als Elutionsmittel chromatographiert. Man erhält 2,5 g 6-Benzyloxy-4-methoxymethyl-1,2,3,4-tetrahydro-β-carbolin-3-carbonsäureisopropylester (65 % Ausbeute als Öl),
oder
eine Suspension von 2,56 g Paraformaldehyd in 8 ml Wasser und 0,8 ml konz. Salzsäure wird bei 80 °C 1 Stunde lang gekocht. 1/10 der daraufhin klaren Lösung a wird nach Abkühlen auf Raumtemperatur zu einer Lösung von 3,8 g (10 mMol) 2-Amino-3-(5-benzyloxyindol-3-yl)-4-methoxybuttersäureisiopropylester in 500 ml Wasser und 10 ml konz. Salzsäure getropft (pH = 3). Nach 1/2 Stunde Rühren wird per Dünnschschichtchromatographie abgeschätzt wieviel Aminoverbindung noch vorhanden ist und entsprechend von der Formaldehydlösung zugegeben. Daraufhin wird noch 1 Stunde gerührt und dann wird mit zweimal 50 ml Toluol ausgeschüttelt. Die organische Phase wird verworfen. Die wässrige Phase wird nach Zufügen von 100 ml Toluol mit 27 %iger Natronlauge auf einen pH von 5,3 eingestellt. Nach Ausschütteln wird noch zweimal mit 50 ml Toluol ausgeschüttelt, diese 3 organischen Phasen zusammengefaßt, über Natriumsulfat getrocknet, filtriert und eingeengt. Man erhält 3,3 g (85%) 6-Benzyloxy-4-methoxymethyl-1,2,3,4-tetrahydro-β-carbolin-3-carbonsäureisopropylester als Öl.

C)

3,3 g (8,5 mMol) 6-Benzyloxy-4-methoxymethyl-1,2,3,4-tetrahydro-β-carbolin-3-carbonsäureisopropylester werden in 150 ml Methylenchlorid gelöst, unter Argon mit 3,9 ml Triäthylamin versetzt und auf - 15 °C gekühlt. Zu dieser Lösung wird bei dieser Temperatur eine Lösung von 3,2 ml (25,6 mMol)t-Butylhypochlorit in 50 ml Methylenchlorid zügig zugetropft. Nach beendeter Zugabe wird 10 Minuten nachgerührt, mit 2,6 ml Triäthylamin versetzt und 2 Stunden bei Raumtemperatur gerührt. Anschließend wird auf die Hälfte eingeengt und einmal mit verdünnter Ammoniaklösung ausgeschüttelt. Die organische Phase wird getrocknet, filtriert und eingeengt. Der Rückstand wird über Kieselgel mit Methylenchlorid : Aceton = 4 : 1 als Elutionsmittel chromatographiert. Nach Umkristallisation aus Essigester erhält man 1,1 g ( 35 % Ausbeute) 6-Benzyloxy-4-methoxymethyl-β-carbolin-3-carbonsäureisopropylester vom Schmelzpunkt 150 - 151 °C.

Beispiel 2

A)

7 g (18 mMol) 6-Benzyloxy-4-methoxymethyl-β-carbolin-3-carbonsäureisopropylester werden in 600 ml Ethanol mit 7 g Palladium/Kohle (10 %) sowie Wasserstoff 8,5 Stunden bei Raumtemperatur und Normaldruck hydriert. Nach Abfiltrieren vom Katalysator wird eingeengt. Man erhält 4,8 g (90 % Ausbeute) 6-Hydroxy-4-methoxymethyl-β-carbolin-3-carbonsäureisopropylester, die ohne weitere Reinigung weiter umgesetzt werden.

B)

500 mg (1,6 mMol) 6-Hydroxy-4-methoxymethyl-β-carbolin-3-carbonsäureisopropylester werden in 50 ml Isopropanol gelöst, mit 500 mg (3,6 mMol) wasserfreiem, gepulvertem Kaliumcarbonat versetzt und unter Argon 10 Minuten gerührt. Anschließend werden 0,25 ml (1,99 mMol) 2Chlorbenzylchlorid zugegeben und die Mischung 2 Stunden zum Rückfluß erhitzt. Nach Absaugen vom Kaliumcarbonat wird das Filtrat eingeengt und über Kieselgel mit Methylenchlorid : Aceton = 3 : 1 als Elutionsmittel getrennt. Man erhält 172 mg 6-(2-Chlorbenzyloxy)-4-methoxymethyl-β-carbolin-3-carbonsäureisopropylester vom Schmelzpunkt 135 - 141 °C.

In analoger Weise werden hergestellt:

6-(4-Chlorbenzyloxy)-4-methoxymethyl-β-carbolin-3-carbonsäureisopropylester vom Schmelzpunkt 184 - 185 °C.

6-(3-Chlorbenzyloxy)-4-methoxymethyl-β-carbolin-3-carbonsäureisopropylester vom Schmelzpunkt 180 - 183 °C.

6-(2-Fluorbenzyloxy)-4-methoxymethyl-β-carbolin-3-carbonsäureisopropylester

6-(3-Fluorbenzyloxy)-4-methoxymethyl-β-carbolin-3-carbonsäureisopropylester vom Schmelzpunkt 168 - 170 °C.

6-(3-Methoxybenzyloxy)-4-methoxymethyl-β-carbolin-3-carbonsäureisopropylester Fp. 160 - 163 °C

6-(4-Cyanobenzyloxy)-4-methoxymethyl-β-carbolin-3-carbonsäureisopropylester Fp. 218 - 222 °C

6-(4-Brombenzyloxy)-4-methoxymethyl-β-carbolin-3-carbonsäureisopropylester Fp. 180 - 190 °C

6-(3-Cyanobenzyloxy)-4-methoxymethyl-β-carbolin-3-carbonsäureisopropylester Fp. 194 - 200 °C

6-(2-Cyanobenzyloxy)-4-methoxymethyl-β-carbolin-3-carbonsäureisopropylester Fp. 168 - 172 °C

6-(2-Bromobenzyloxy)-4-methoxymethyl-β-carbolin-3-carbonsäureisopropylester Fp. 148 - 151 °C

6-(4-Fluorbenzyloxy)-4-methoxymethyl-β-carbolin-3-carbonsäureisopropylester vom Schmelzpunkt 160 -

163 °C.
6(2,4-Dichlorbenzyloxy)-4-methoxymethyl-β-carbolin-3-carbonsäureisopropylester vom Schmelzpunkt 156 - 159 °C.
6(4-Methylbenzyloxy)-4-methoxymethyl-β-carbolin-3-carbonsäureisopropylester
6-(3-Methylbenzyloxy)-4-methoxymethyl-β-carbolin-3-carbonsäureisopropylester
6-(2-Methylbenzyloxy)-4-methoxymethyl-β-carbolin-3-carbonsäureisopropylester.

Beispiel 3

1,4 g (3,6 mMol) 6-Benzyloxy-4-methoxymethyl-β-carbolin-3-carbonsäureethylester werden in 100 ml i-Propanol mit 0,7 ml (2,2 mMol) Titantetraisopropoxyd 2 Stunden am Rückfluß gekocht. Nach Einengen wird in 80 ml 1n Salzsäure aufgenommen und mit 250 ml Essigester ausgeschüttelt. Die Essigesterphase wird mit wenig Wasser gewaschen, getrocknet, filtriert und eingeengt. Nach einer Chromatographie über Kieselgel mit Methylenchlorid : Aceton = 4 : 1 als Elutionsmittel erhält man in 80 % Ausbeute 6-Benzyloxy-4-methoxymethyl-β-carbolin-3-carbonsäureisopropylester vom Schmelzpunkt 150 - 151 °C.

In grundsätzlich analoger Weise, nur unter Einsatz des entsprechenden Alkohols werden hergestellt:
6-Benzyloxy-4-methoxymethyl-β-carbolin-3-carbonsäurecyclopentylester.
6-Benzyloxy-4-methoxymethyl-β-carbolin-3-carbonsäuremethylcyclopropylester.
6-Benzyloxy-4-methoxymethyl-β-carbolin-3-carbonsäurecyclohexylester Fp. 177 °C
6-(3-Fluorbenzyloxy)-4-methoxymethyl-β-carbolin-3-carbonsäure-2-butylester Fp. 145 °C
6-Benzyloxy-4-methoxymethyl-β-carbolin-3-carbonsäureneopentylester Fp. 192 °C
6-Benzyloxy-4-methoxymethyl-β-carbolin-3-carbonsäureisobutylester Fp. 157 - 161 °C
6-Benzyloxy-4-methoxymethyl-β-carbolin-3-carbonsäure-2-butylester vom Schmelzpunkt 119 - 123 °C.

Beispiel 4

1 g 6-Benzyloxy-4-methoxymethyl-β-carbolin-3-carbonsäure werden in 10 ml Aminalester 3,5 Stunden auf eine Badtemperatur von 120 °C erhitzt. Nach Eindampfen wird der Rückstand über Kieselgel mit Hexan : Aceton = 13 : 7 als Elutionsmittel chromatographiert. Man erhält 300 mg 6-Benzyloxy-4-methoxymethyl-β-carbolin-3-carbonsäure-t-butylester

In analoger Weise wird hergestellt:
6-(4-Fluorbenzyloxy)-4-methoxymethyl-β-carbolin-3-carbonsäure-t-butylester Fp. 110 - 167 °C

Beispiel 5

A)

78,1 g 5-Benzyloxy-4-methoxymethyl-β-carbolin-3-carbonsäureethylester werden unter Argon in 1 Liter Isopropanol suspendiert, mit 28 ml Titantetraisoproxid versetzt und 4 Stunden am Rückfluß erhitzt. Nach Abkühlen, Eindampfen und Chromatographie über Kieselgel mit Hexan : Aceton = 1 : 2 als Elutionsmittel werden Verunreinigungen abgetrennt. Beim Übergang auf Hexan : Aceton = 1 : 3 und Hexan : i-Propanol = 3,5 : 1 werden 56,1 g 5-Benzyloxy-4-methoxymethyl-β-carbonsäureisopropylester vom Schmelzpunkt 208 - 209 °C isoliert.

In analoger Weise werden hergestellt:
5-(3-Chlorbenzyloxy)-4-methoxymethyl-β-carbolin-3-carbonsäureisopropylester vom Schmelzpunkt 173 - 174 °C
5-(3-Fluorbenzyloxy)-4-methoxymethyl-β-carbolin-3-carbonsäureisopropylester vom Schmelzpunkt 181 - 182 °C
5-(2-Fluorbenzyloxy)-4-methoxymethyl-β-carbolin-3-carbonsäureisopropylester vom Schmelzpunkt 145 - 146 °C
5-(4-Chlor-benzyloxy)-4-methoxymethyl-β-carbolin-3-carbonsäureisopropylester vom Schmelzpunkt 212 - 213 °C.
5-(2-Chlorbenzyloxy)-4-methoxymethyl-β-carbolin-3-carbonsäureisopropylester vom Schmelzpunkt 198 - 199 °C.

5-(4-Fluorbenzyloxy)-4-methoxymethyl-β-carbolin-3-carbonsäureisopropylester.
5-(3-Methylbenzyloxy)-4-methoxymethyl-β-carbolin-3-carbonsäureisopropylester.

In der zu A) grundsätzlich analogen Art wurden unter Verwendung der entsprechenden Alkohole hergestellt:
5-Benzyloxy-4-methoxymethyl-β-carbolin-3-carbonsäurecyclohexylester vom Schmelzpunkt 143 - 145 °C.
5-Benzyloxy-4-methoxymethyl-β-carbolin-3-carbonsäurehexafluorisopropylester.
5-Benzyloxy-4-methoxymethyl-β-carbolin-3-carbonsäuremethylcyclopropylester.
5-Benzyloxy-4-methoxymethyl-β-carbolin-3-carbonsäureisobutylester.
5-(4-Chlorbenzyloxy)-4-methoxymethyl-β-carbolin-3-carbonsäurecyclopentylester.

B)

740 mg (2 mMol) 5-Benzyloxy-4-methoxymethyl-β-carbolin-3-carbonsäure werden in 50 ml Ethanol und 20 ml Wasser mit 977 mg Cäsiumkarbonat 2 Stunden bei 80 °C gerührt. Nach Einengen am Rotationsverdampfer und Trocknen im Exsikkator wird in 50 ml DMF aufgenommen mit 0,2 ml 2-Brompropan versetzt und 8 Stunden auf 60 - 70 °C erwärmt. Nach Einengen wird über Kieselgel mit Hexan : Aceton = 1 : 1 als Elutionsmittel chromatographiert und man erhält 340 mg 5-Benzyloxy-4-methoxymethyl-β-carbolin-3-carbonsäureisopropylester von o.g. Schmelzpunkt.

In analoger Weise werden hergestellt:
5(3-Chlorbenzyloxy)-4-methoxymethyl-β-carbolin-3-carbonsäurecyclobutylester vom Schmelzpunkt 166 - 167 °C
5(3-Chlorbenzyloxy)-4-methoxymethyl-β-carbolin-3-carbonsäurecyclopropylester vom Schmelzpunkt 167 - 178 °C
5(3-Chlorbenzyloxy)-4-methoxymethyl-β-carbolin-3-carbonsäureisobutylester

Beispiel 6

500 mg 5-Benzyloxy-4-methoxymethyl-β-carbolin-3-carbonsäure werden mit 5 ml Aminalester 3,5 Stunden auf 120 °C Badtemperatur erwärmt. Nach Eindampfen zur Trockene wird der Rückstand über Kieselgel mit Hexan : Aceton = 13 : 7 als Elutionsmittel chromatographiert. Man erhält 190 mg 5-Benzyloxy-4-methoxymethyl-β-carbolin-3-carbonsäure-t-butylester vom Schmelzpunkt 180 - 181 °C.

In analoger Weise wird hergestellt:
5-(3-Benzyloxy)-4-methoxymethyl-β-carbolin-3-carbonsäure-t-butylester

## Ansprüche

**1.** 5- oder 6- substituierten β-Carbolin-3-carbonsäureester der allgemeinen Formel I

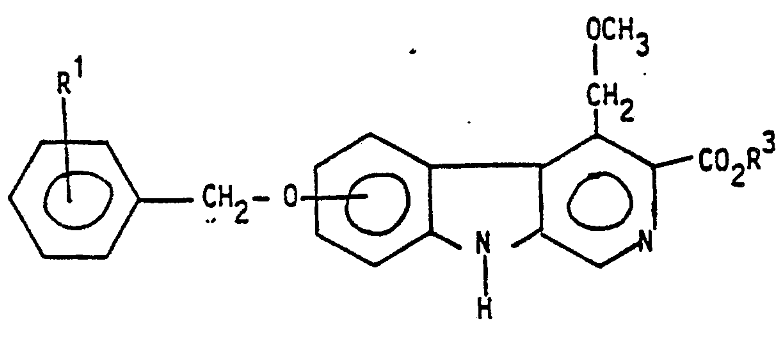

I

worin

- $R^1$ Wasserstoff, Nitrilo, Halogen, $C_{1-4}$-Alkyl, $C_{1-4}$-Alkoxy bedeutet und
- $R^3$ eine verzweigte $C_{3-6}$-Alkylgruppe ist, die gegebenenfalls mit Halogen substituiert ist oder eine $C_{3-6}$-Cycloalkylgruppe, die gegebenenfalls methylsubstituiert ist und
- $R^1$ eine- oder mehrfach stehen kann.

**2.** 6-(2-Chlor-benzyloxy)-4-methoxymethyl-β-carbolin-3-carbonsäureisopropylester,

6-(4-Chlorbenzyloxy)-4-methoxymethyl-β-carbolin-3-carbonsäureisopropylester,

6-(3-Chlorbenzyloxy)-4-methoxymethyl-β-carbolin-3-carbonsäureisopropylester,

6-(2-Fluorbenzyloxy)-4-methoxymethyl-β-carbolin-3-carbonsäureisopropylester,

6-(3-Fluorbenzyloxy)-4-methoxymethyl-β-carbolin-3-carbonsäureisopropylester,

6-(4-Fluorbenzyloxy)-4-methoxymethyl-β-carbolin-3-carbonsäureisopropylester,

6-(2,4-Dichlorbenzyloxy-4-methoxymethyl-β-carbolin-3-carbonsäureisopropylester,

6-(4-Methylbenzyloxy)-4-methoxymethyl-β-carbolin-3-carbonsäureisopropylester,

6-(3-Methylbenzyloxy)-4-methoxymethyl-β-carbolin-3-carbonsäureisopropylester,

6-(2-Methylbenzyloxy)-4-methoxymethyl-β-carbolin-3-carbonsäureisopropylester,

6-Benzyloxy-4-methoxymethyl-β-carbolin-3-carbonsäureisopropylester,

6-Benzyloxy-4-methoxymethyl-β-carbolin-3-carbonsäurecyclopentylester,

6-Benzyloxy-4-methoxymethyl-β-carbolin-3-carbonsäuremethylcyclopropylester,

6-Benzyloxy-4-methoxymethyl-β-carbolin-3-carbonsäure-2-butylester,

6-Benzyloxy-4-methoxymethyl-β-carbolin-3-carbonsäure-t-butylester,

5-Benzyloxy-4-methoxymethyl-β-carbolin-3-carbonsäureisopropylester,
5-(4-Chlor-benzyloxy)-4-methoxymethyl-β-carbolin-3-carbonsäureisopropylester,

5-(2-Chlorbenzyloxy)-4-methoxymethyl-β-carbolin-3-carbonsäureisopropylester,

5-(4-Fluorbenzyloxy)-4-methoxymethyl-β-carbolin-3-carbonsäureisopropylester,

5-(3-Methylbenzyloxy)-4-methoxymethyl-β-carbolin-3-carbonsäureisopropylester,

5-Benzyloxy-4-methoxymethyl-β-carbolin-3-carbonsäurecyclohexylester,

5-Benzyloxy-4-methoxymethyl-β-carbolin-3-carbonsäurehexafluorisopropylester,

5-Benzyloxy-4-methoxymethyl-β-carbolin-3-carbonsäuremethylcyclopropylester,

5-Benzyloxy-4-methoxymethyl-β-carbolin-3-carbonsäureisobutylester,

5-(4-Chlorbenzyloxy)-4-methoxymethyl-β-carbolin-3-carbonsäurecyclopentylester,

5-Benzyloxy-4-methoxymethyl-β-carbolin-3-carbonsäure-t-butylester nach Anspruch 1.

**3.** Verfahren zur Herstellung der Verbindungen der allgemeinen Formel I dadurch, daß man
a) eine Verbindung der allgemeinen Formel II

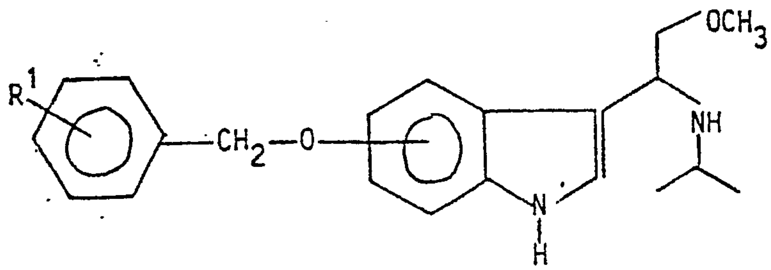

II,

worin

$R^1$ die oben genannte Bedeutung hat, mit dem entsprechenden Ester eines Glycinimins der allgemeinen Formel III

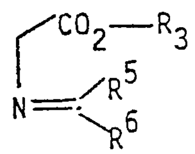

III,

worin

$R^3$ die oben genannte Bedeutung hat, worin $R^5$ ein vorzugweise durch 4-Methoxy oder Wasserstoff substituierter Aromat und $R^6$ Wasserstoff oder gegebenenfalls ein Aromat ist, umsetzt und anschließend das Imin hydrolytisch zum Amin der allgemeinen Formel IV

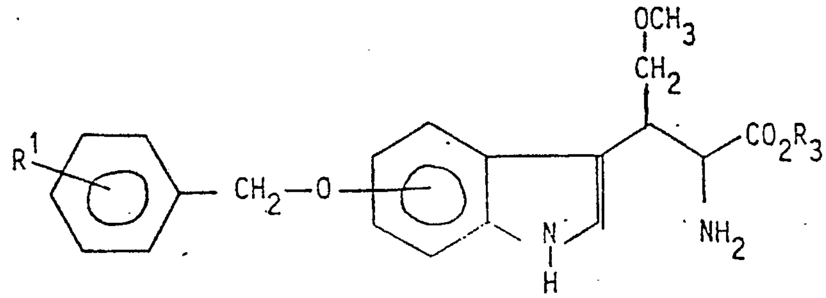

IV,

spaltet, mit Formaldehyd oder Glyoxylsäure cyclisiert, wobei die Cyclisierung auch grundsätzlich mit der sauren Spaltung des Imins als Eintopfreaktion verbunden werden kann, und anschließend aromatisiert und gewünschtenfalls die Benzylgruppe abspaltet und die so erhaltene frei Hydroxygruppe anschließend veräthert mit = einer Verbindung der allgemeinen Formel V

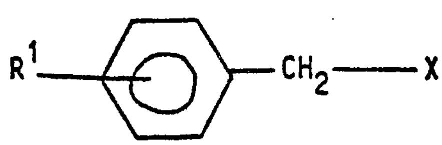

V,

worin

$R^1$ die oben genannte Bedeutung hat und X Halogen, Mesyl oder Tosyl bedeutet oder

b) eine Verbindung der allgemeinen Formel VI

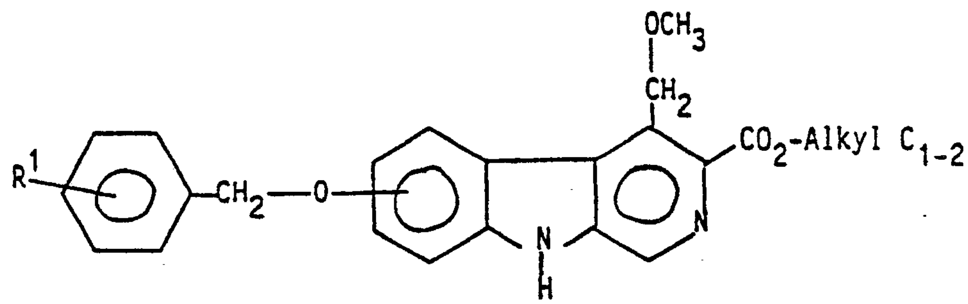

VI,

worin $R^1$ die oben genannte Bedeutung hat, umestert.

4. Verwendung der Verbindungen der allgemeinen Formel I als Pharmazeutika.

5. Verwendung der Verbindungen nach Anspruch 1 und 2 zur Herstellung eines Arzneimittels zur Behandlung von Krankheiten des Zentralnervensystems.

6. Arzneimittel auf Basis der Verbindungen nach den Ansprüchen 1 und 2.

## Claims

1. 5- or 6-substituted β-carboline-3-carboxylic acid esters of the general formula I

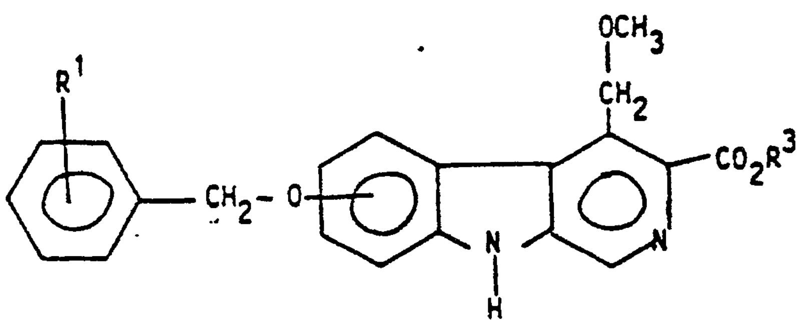

I

in which

$R^1$ represents hydrogen, nitrilo, halogen, $C_{1-4}$-alkyl or $C_{1-4}$-alkoxy and

$R^3$ is a branched $C_{3-6}$-alkyl group that is optionally substituted by halogen, or is a $C_{3-6}$-cycloalkyl group that is optionally substituted by methyl, and there may be one or more radicals $R^1$.

2. 6-(2-Chlorobenzyloxy)-4-methoxymethyl-β-carboline-3-carboxylic acid isopropyl ester,

6-(4-chlorobenzyloxy)-4-methoxymethyl-β-carboline-3-carboxylic acid isopropyl ester,

6-(3-chlorobenzyloxy)-4-methoxymethyl-β-carboline-3-carboxylic acid isopropyl ester,

6-(2-fluorobenzyloxy)-4-methoxymethyl-β-carboline-3-carboxylic acid isopropyl ester,

6-(3-fluorobenzyloxy)-4-methoxymethyl-β-carboline-3-carboxylic acid isopropyl ester,

6-(4-fluorobenzyloxy)-4-methoxymethyl-β-carboline-3-carboxylic acid isopropyl ester,

6-(2,4-dichlorobenzyloxy)-4-methoxymethyl-β-carboline-3-carboxylic acid isopropyl ester,

6-(4-methylbenzyloxy)-4-methoxymethyl-β-carboline-3-carboxylic acid isopropyl ester,

6-(3-methylbenzyloxy)-4-methoxymethyl-β-carboline-3-carboxylic acid isopropyl ester,

6-(2-methylbenzyloxy)-4-methoxymethyl-β-carboline-3-carboxylic acid isopropyl ester,

6-benzyloxy-4-methoxymethyl-β-carboline-3-carboxylic acid isopropyl ester,

6-benzyloxy-4-methoxymethyl-β-carboline-3-carboxylic acid cyclopentyl ester,

6-benzyloxy-4-methoxymethyl-β-carboline-3-carboxylic acid methylcyclopropyl ester,

6-benzyloxy-4-methoxymethyl-β-carboline-3-carboxylic acid 2-butyl ester,

6-benzyloxy-4-methoxymethyl-β-carboline-3-carboxylic acid t.-butyl ester,

5-benzyloxy-4-methoxymethyl-β-carboline-3-carboxylic acid isopropyl ester,

5-(4-chlorobenzyloxy)-4-methoxymethyl-β-carboline-3-carboxylic acid isopropyl ester,

5-(2-chlorobenzyloxy)-4-methoxymethyl-β-carboline-3-carboxylic acid isopropyl ester,

5-(4-fluorobenzyloxy)-4-methoxymethyl-β-carboline-3-carboxylic acid isopropyl ester,

5-(3-methylbenzyloxy)-4-methoxymethyl-β-carboline-3-carboxylic acid isopropyl ester,

5-benzyloxy-4-methoxymethyl-β-carboline-3-carboxylic acid cyclohexyl ester,

5-benzyloxy-4-methoxymethyl-β-carboline-3-carboxylic acid hexafluoroisopropyl ester,

5-benzyloxy-4-methoxymethyl-β-carboline-3-carboxylic acid methylcyclopropyl ester,

5-benzyloxy-4-methoxymethyl-β-carboline-3-carboxylic acid isobutyl ester,

5-(4-chlorobenzyloxy)-4-methoxymethyl-β-carboline-3-carboxylic acid cyclopentyl ester,

5-benzyloxy-4-methoxymethyl-β-carboline-3-carboxylic acid t.-butyl ester, according to claim 1.

**3.** A process for the manufacture of compounds of the general formula I, characterised in that
a) a compound of the general formula II

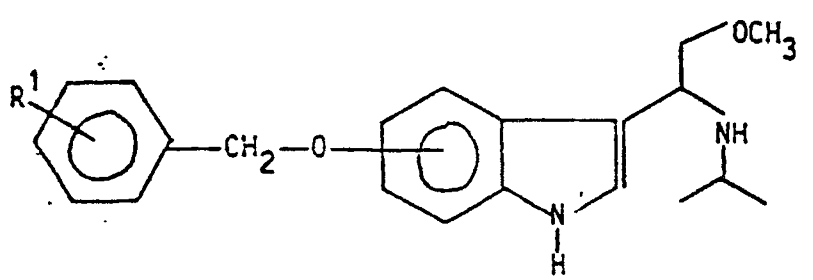

II

in which
$R^1$ has the meaning given hereinbefore, is reacted with the corresponding ester of a glycinimine of the general formula III

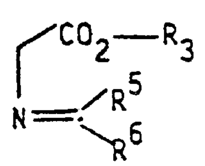

III

in which
$R^3$ has the meaning given hereinbefore, wherein $R^5$ is an aromatic radical preferably substituted by 4-methoxy or hydrogen, and $R^6$ is hydrogen or optionally an aromatic radical, and then the imine is cleaved by hydrolysis to form the amine of the general formula IV

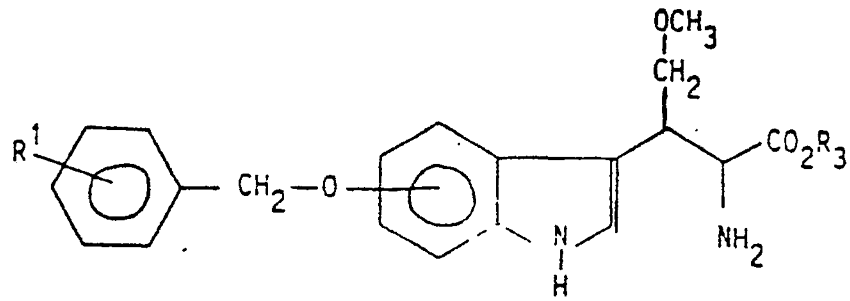

IV ,

cyclised with formaldehyde or glyoxylic acid, it also being possible, in principle, for the cyclisation to be combined with the acid cleavage of the imine as a one-pot reaction, and then aromatized and, if desired, the benzyl group is removed, and the free hydroxy group so obtained is then etherified with a compound of the general formula V

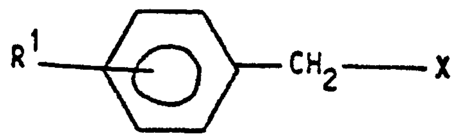

V,

in which
$R^1$ has the meaning given hereinbefore and X represents halogen, mesyl or tosyl, or
b) a compound of the general formula VI

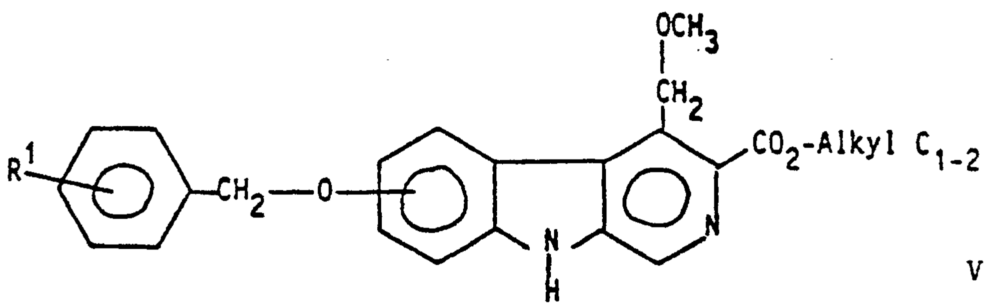

VI

wherein $R^1$ has the meaning given hereinbefore, is subjected to ester interchange.

4. Use of the compounds of the general formula I as pharmaceutical agents.

5. Use of the compounds according to claims 1 and 2 for the preparation of a medicament for the treatment of diseases of the central nervous system.

6. Medicament based on the compounds according to claims 1 and 2.

## Revendications

1. Esters d'acides $\beta$-carboline-carboxyliques-3 substitués en 5 ou en 6 qui répondent à la formule générale I :

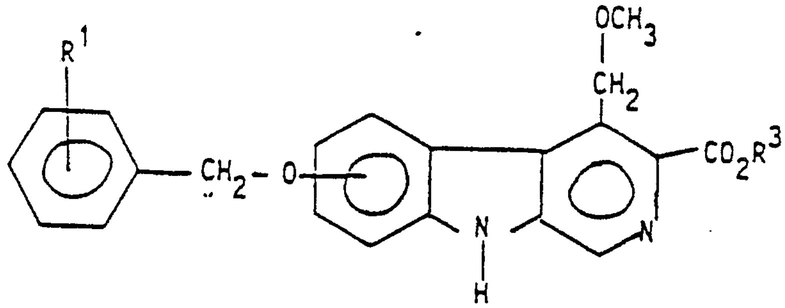 I

dans laquelle

$R^1$ représente l'hydrogène, un radical cyano, un halogène, un alkyle en $C_{1-4}$ ou un alcoxy en $C_{1-4}$ et

$R^3$ représente un alkyle en $C_{3-6}$ ramifié qui porte éventuellement un halogène, ou représente un cycloalkyle en $C_{3-6}$ éventuellement porteur d'un méthyle,

et dans laquelle le substituant R1 peut exister en un ou en plusieurs exemplaires.

**2.** Composés selon la revendication 1, en l'espèce :

le (chloro-2 benzyloxy)-6 méthoxyméthyl-4 β-carbolinecarboxylate-3 d'isopropyle,

le (chloro-4 benzyloxy)-6 méthoxyméthyl-4 β-carbolinecarboxylate-3 d'isopropyle,

le (chloro-3 benzyloxy)-6 méthoxyméthyl-4 β-carbolinecarboxylate-3 d'isopropyle,

le (fluoro-2 benzyloxy)-6 méthoxyméthyl-4 β-carbolinecarboxylate-3 d'isopropyle,

le (fluoro-2 benzyloxy)-6 méthoxyméthyl-4 β-carbolinecarboxylate-3 d'isopropyle,

le (fluoro-4 benzyloxy)-6 méthoxyméthyl-4 β-carbolinecarboxylate-3 d'isopropyle,

le (dichloro-2,4 benzyloxy)-6 méthoxyméthyl-4 β-carbolinecarboxylate-3 d'isopropyle,

le (méthyl-4 benzyloxy)-6 méthoxyméthyl-4 β-carbolinecarboxylate-3 d'isopropyle,

le (méthyl-3 benzyloxy)-6 méthoxyméthyl-4 β-carbolinecarboxylate-3 d'isopropyle,

le (méthyl-2 benzyloxy)-6 méthoxyméthyl-4 β-carbolinecarboxylate-3 d'isopropyle,

le benzyloxy)-6 méthoxyméthyl-4 β-carboline-carboxylate-3 d'isopropyle,

le benzyloxy-6 méthoxyméthyl-4 β-carboline-carboxylate-3 de cyclopentyle,

le benzyloxy-6 méthoxyméthyl-4 β-carboline-carboxylate-3 de méthylcyclopropyle,

le benzyloxy-6 méthoxyméthyl-4 β-carboline-carboxylate-3 de méthyl-1 propyle,

le benzyloxy-6 méthoxyméthyl-4 β-carboline-carboxylate-3 de tert-butyle,

le benzyloxy-5 méthoxyméthyl-4 β-carboline-carboxylate-3 d'isopropyle,

le (chloro-4 benzyloxy)-5 méthoxyméthyl-4 β-carbolinecarboxylate-3 d'isopropyle,

le (chloro-2 benzyloxy)-5 méthoxyméthyl-4 β-carbolinecarboxylate-3 d'isopropyle,

le (fluoro-4 benzyloxy)-5 méthoxyméthyl-4 β-carbolinecarboxylate-3 d'isopropyle,

le (méthyl-3 benzyloxy)-5 méthoxyméthyl-4 β-carbolinecarboxylate-3 d'isopropyle,

le benzyloxy-5 méthoxyméthyl-4 β-carboline-carboxylate-3 de cyclohexyle,

le benzyloxy-5 méthoxyméthyl-4 β-carboline-carboxylate-3 d'hexafluoro-isopropyle,

le benzyloxy-5 méthoxyméthyl-4 β-carboline-carboxylate-3 de méthylcyclopropyle,

le benzyloxy-5 méthoxyméthyl-4 β-carboline-carboxylate-3 d'isobutyle,

le (chloro-4 benzyloxy)-5 méthoxyméthyl-4 β-carbolinecarboxylate-3 de cyclopentyle,

le benzyloxy-5 méthoxyméthyl-4 β-carboline-carboxylate-3 de tert-butyle.

**3.** Procédé pour préparer les composés de formule générale I, procédé caractérisé en ce que :
a) On fait réagir un composé répondant à la formule générale II :

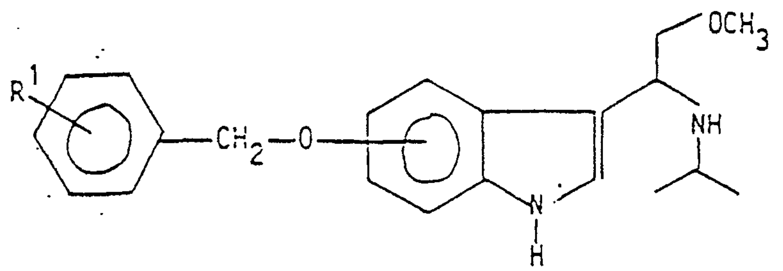

II

dans laquelle $R^1$ a la signification qui lui a été donnée ci-dessus,
avec l'ester correspondant d'une glycine-imine répondant à la formule générale III :

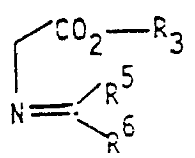

III

dans laquelle $R^3$ a la signification qui lui a été donnée ci-dessus, $R^5$ représente un radical aromatique dans lequel il y a de préférence un hydrogène ou un radical méthoxy à la position 4, et $R^6$ représente l'hydrogène ou, éventuellement, un radical aromatique, puis on coupe l'imine par hydrolyse pour la convertir en l'amine de formule générale IV :

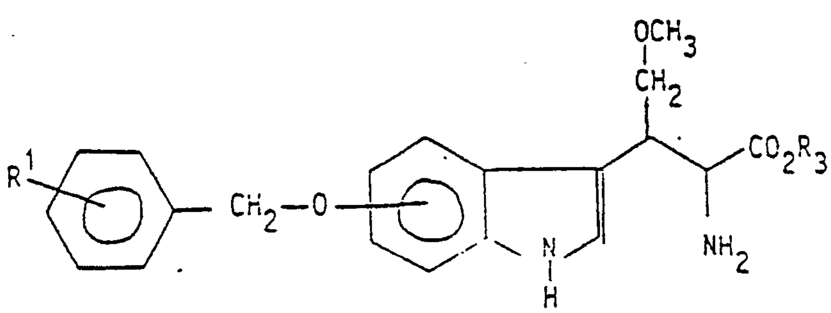

IV,

on cyclise au moyen du formaldéhyde ou de l'acide glyoxylique, la cyclisation pouvant en principe aussi être liée à la coupure acide de l'imine sous la forme d'une réaction en un seul pot, après quoi on aromatise et, si on le désire, on coupe le radical benzyle, puis estérifie le radical hydroxy libre ainsi obtenu au moyen d'un composé répondant à la formule générale V :

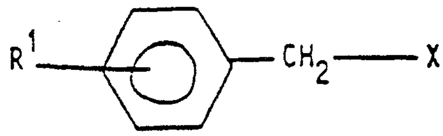

V

dans laquelle $R^1$ a la signification qui lui a été donnée ci-dessus et X représente un halogène ou un radical mésyle ou tosyle,
ou
b) on transestérifie un composé répondant à la formule générale VI :

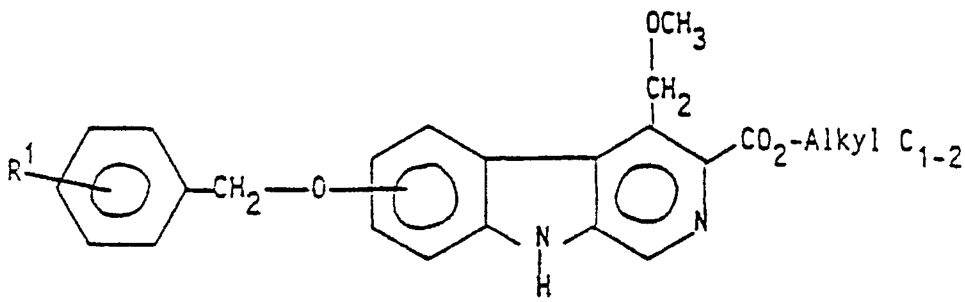

VI

dans laquelle $R^1$ a la signification qui lui a été donnée ci-dessus.

**4.** Application des composés de formule générale I comme produits pharmaceutiques.

**5.** Application des composés selon l'une des revendications 1 et 2 à la fabrication d'un médicament pour le traitement de maladies du système nerveux central.

**6.** Médicament à base de composés selon l'une des revendications 1 et 2.